# EUROPEAN PATENT APPLICATION

(11) **EP 3 714 917 A1**
(43) Date of publication of application: **30.09.2020**
(21) Application number: 20161523.4
(22) Date of filing: 06.03.2020
(51) Int. Cl.: A61M 1/16, A61M 1/36, A61L 2/18

(54) **SERVICE SYSTEM, FOR EXTRACORPOREAL CIRCULATION APPARATUSES, AND CORRESPONDING METHOD FOR PREVENTING THE PROLIFERATION OF BACTERIAL POPULATION**

(30) Priority: 25.03.2019 IT 201900004237
(71) Applicant: Tecnoline S.p.A., 41033 Concordia Sulla Secchia, MO (IT)
(72) Inventor: PROVASI, Stefano, 41033 CONCORDIA SULLA SECCHIA MO (IT); ALBORESI, Gino, 41037 MIRANDOLA MO (IT)
(74) Representative: Modiano, Micaela Nadia

(57) **Abstract**

A service system, for extracorporeal circulation apparatuses, which comprises at least: a heat exchanger (2), which can be passed through by blood destined for extracorporeal circulation and by a service liquid (A) which is adapted to heat and/or cool the blood, a tank (3) for containing the service liquid (A), a delivery branch (4), which is normally interposed between the tank (3) and the exchanger (2), and a return branch (6), which is normally interposed between the exchanger (2) and the tank (3). The exchanger (2), the tank (3) and the branches (4, 6) form a service circuit (7a), which is normally passed through by the service liquid (A).

The system comprises a unit (8) for dispensing ozone and a filtering assembly (9), which comprises a tubular body (10) which can be connected to the tank (3) through the branches (4, 6) as a temporary replacement for the exchanger (2), to provide a temporary disinfection circuit (7b). The body (10) is affected by at least one filter (11) for the service liquid (A) and by a connector (12) for mixing the liquid (A) with the ozone dispensed by the unit (8).

## Description

The present invention relates to a service system, for extracorporeal circulation apparatuses, and a corresponding method for preventing the proliferation of bacterial population.

As is known, in nature numerous bacteria exist which normally are harmless to humans, but which under certain conditions, and in individuals who for various reasons are ill, can instead be dangerous or even lethal.

One of these is certainly the mycobacterium chimaera, which normally lives in the earth, in water and in plumbing systems, but which is also able to easily move and prosper even in the atmosphere.

The presence of the mycobacterium in the water of plumbing systems, and therefore in the water that reaches our homes, is no cause for concern because it is an organism that is normally not capable of causing any harm to human beings. But when the bacterium infests the water in hospitals, it can represent a serious threat to health, because it comes into contact with immune-compromised persons and/or with body tissues and organs other than those involved when it is simply ingested with water.

More specifically, it has been found that this bacterium constitutes a severe threat to persons who undergo cardiac surgery, for whom, in order to provide a temporary extracorporeal circulation, use is made of machines for heating and cooling the blood, and these machines can be colonized by the bacterium.

Such machines in fact have circuits that are passed through by water (used to exchange heat with the blood), which can contain the chimaera bacterium. Owing to the stagnation of water vapor in ducts and/or as a consequence of the fall of water droplets in the operating theater (for example during the steps of preparation or cleaning of the machine), the bacterium can spread in the operating theater and/or infect the patient, in particular the cardiac valves, where it can lie dormant for several years and then flare up, causing extremely serious inflammatory diseases which can have a lethal outcome.

In light therefore of the hazardous nature of the situation described above, the companies that make the machines have studied various different ways of preventing these infections, but with unsatisfactory results.

Such methods in fact comprise requiring the daily change of the water that passes through the circuits and/or the frequent use of chemical disinfectants, to be mixed with the water. However, these solutions are very laborious, as they involve an unwanted expenditure of time and, where they use chemical disinfectants, they also have other contraindications.

The aim of the present invention is to solve the above mentioned problems, by providing a service system that ensures practical means of preventing infections by mycobacterium chimaera.

It is also the aim of the present invention to solve the above mentioned problems, by providing a prevention method that provides practical means for preventing infections by mycobacterium chimaera and of preventing the proliferation of bacterial population in general.

Within this aim, an object of the invention is to provide a system and/or propose a method that makes it possible to sanitize and disinfect, simply and completely, circuits and machines involved in extracorporeal circulation.

Another object of the invention is to provide a system that ensures a high reliability of operation and which can be easily implemented, starting with elements and materials readily available on the market.

Another object of the invention is to provide a system that adopts an alternative technical and structural architecture to those of conventional systems.

Another object of the invention is to provide a method that can be easily implemented starting with elements and materials readily available on the market.

Another object of the invention is to provide a system and/or a method that are of low cost and safely applied.

This aim and these and other objects which will become better apparent hereinafter are achieved by a system according to claim 1, a kit according to claim 7 and a method according to claim 8.

Further characteristics and advantages of the invention will become better apparent from the description of two preferred, but not exclusive, embodiments of the system and the method according to the invention, which are illustrated by way of non-limiting example in the accompanying drawings wherein:
Figure 1 is a simplified diagram of the system according to the invention;

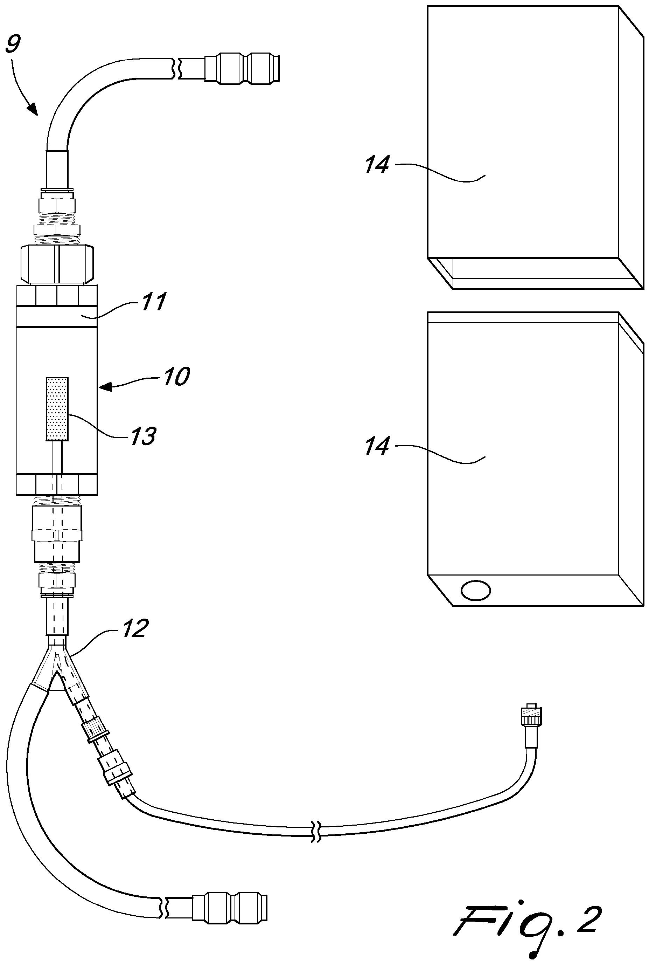

Figure 2 is a perspective view of a first embodiment of a filtering assembly, comprised in the system according to the invention;

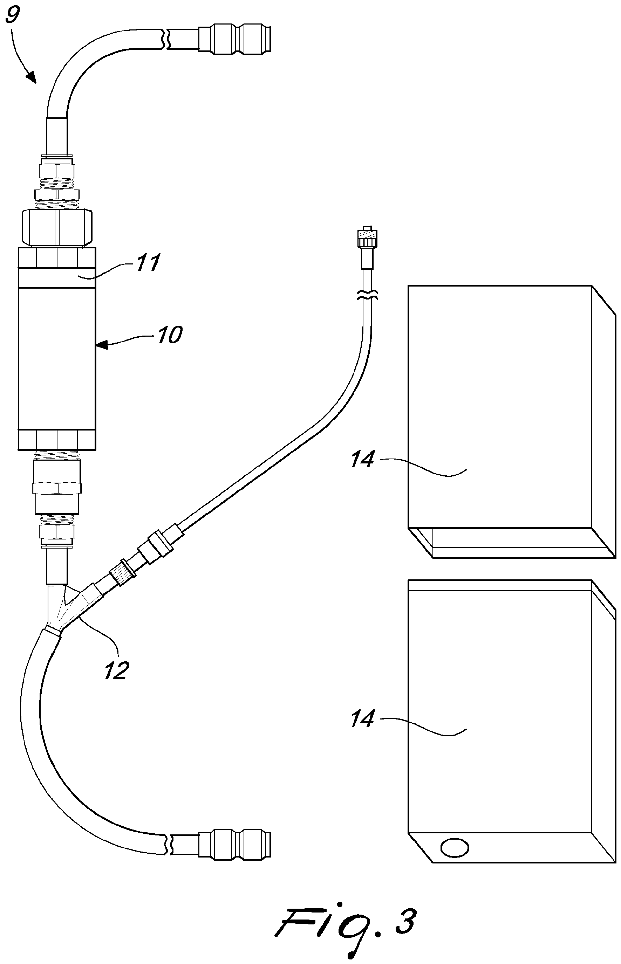

Figure 3 is a perspective view of a second embodiment of a filtering assembly, comprised in the system according to the invention;

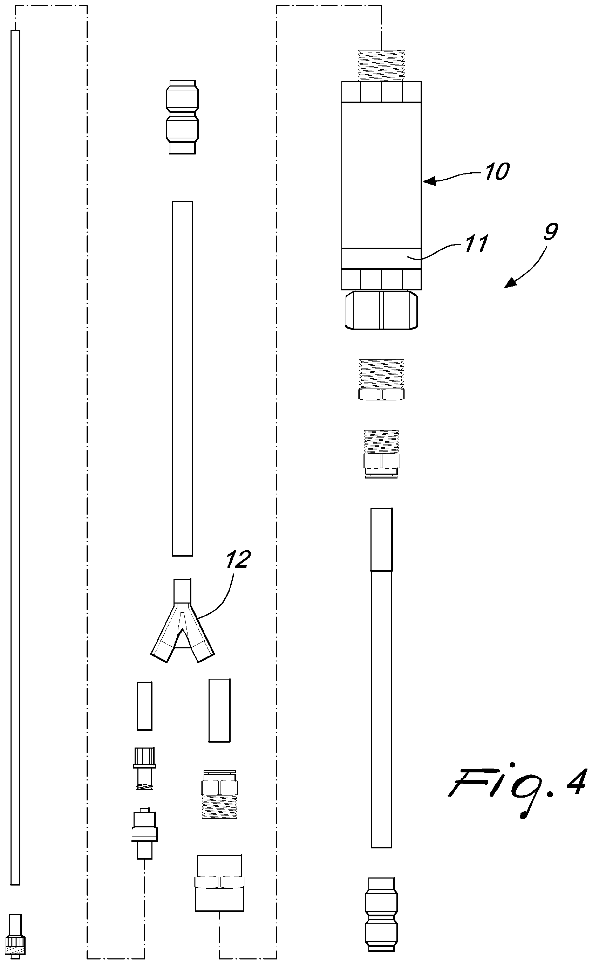

Figure 4 is an exploded view of some components of the assembly of Figure 3.

With particular reference to the figures, the reference numeral 1 generally designates a service system, which can be implemented and/or used for extracorporeal circulation apparatuses (which are conventional), which may be already present in operating theaters.

As is known, in the known art, and in the present discussion, extracorporeal circulation apparatuses (in which the invention is used) are biomedical machines, used mostly in cardiac surgery to ensure the survival of the patient during the operation, temporarily standing in for the patient's cardiopulmonary functions.

The system 1 therefore comprises at least one heat exchanger 2, which is passed through (typically, but not exclusively, cyclically) by blood destined for extracorporeal circulation, and by a service liquid A, and which has the task of heating and/or cooling that blood (circulating within the exchanger 2), as a function of the specific requirements and surgical necessities.

It should be noted in this regard that the service liquid A is typically water (optionally with additives of various kinds), although the possibility is not ruled out of using other liquids A, while remaining within the scope of protection claimed herein.

The system 1 further comprises at least one tank 3 for containing the service liquid A.

In addition, the system 1 comprises a delivery branch 4 which is normally interposed between the tank 3 and the exchanger 2, in order to ensure the liquid A is sent to the exchanger 2 (typically, by way of a pump 5).

The system 1 likewise comprises a return branch 6, which is normally interposed between the exchanger 2 and the tank 3, in order to allow the return of the liquid A to the tank 3.

It should be noted that the term "normally" is used here with reference to the condition that arises during the operation of the extracorporeal circulation apparatus, i.e. when the liquid A heats or cools the blood.

So in fact, the exchanger 2, the tank 3 and the branches 4, 6 (interposed between the exchanger 2 and the tank 3) form a service circuit 7a, which is normally passed through by the service liquid A (typically cyclically).

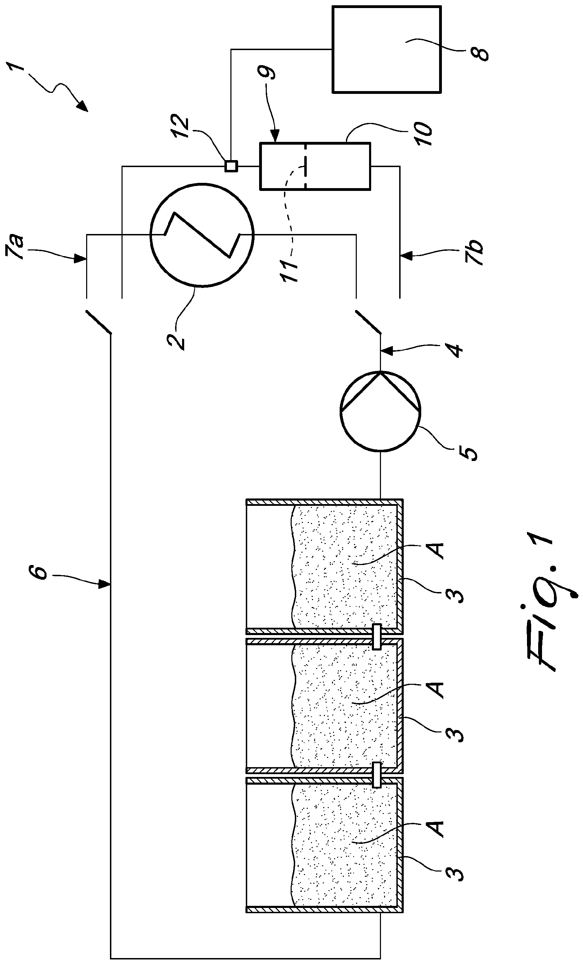

It must be noted that the service circuit 7a (and more generally the system 1) can comprise a number at will of tanks 3 (for example three, as in Figure 1), which are mutually connected in series or in parallel and which in any case are interposed between the branches 4, 6).

In any case, up to this point the system 1 and the service circuit 7a defined above are substantially conventional, and one of the objectives of the invention is in fact to be able to intervene easily in existing solutions as well, or in any case intervening in line with existing solutions, in any case ensuring an effective prevention of chimaera bacterium infections, and more besides.

According to the invention, the system 1 comprises a unit 8 for dispensing ozone, which can be of the commercial type (supplied with a bottle of oxygen for example) or specially made, according to the specific requirements.

In the latter case, in a preferred embodiment, which however does not limit the application of the invention, the ozone generation unit 8 comprises an oxygen enrichment device, for subsequently generating ozone starting from atmospheric air.

Furthermore, according to the invention the system 1 comprises a filtering assembly 9, which in turn comprises a tubular body 10 which can be connected to the tank 3 (or to the tanks 3) through the branches 4, 6 as a temporary replacement for the exchanger 2 (i.e., having previously taken care to disconnect the branches 4, 6 from the latter), in order to provide a temporary circuit 7b, for disinfection. The temporary circuit 7b comprises the assembly 9 (the body 10), the tank 3 and the branches 4, 6. The body 10 is affected by at least one filter 11 for the service liquid A and by a connector 12 for mixing the liquid A with the ozone dispensed by the unit 8.

After having temporarily isolated the exchanger 2 (by substituting it with the filtering assembly 9), it is then possible to send ozone mixed with water (or other service liquid A) into the temporary circuit 7b (which is made up mostly of the same components as the service circuit 7a). The mixture of liquid A and ozone thus flows through the branches 4, 6 and reaches the tanks 3, subjecting such components, and obviously the liquid A, to the beneficial properties of ozone, which as is known has an intense oxidant power and is capable of having a broad-spectrum antibacterial, antiviral and antimycotic action. So by using the system 1 and in particular by periodically mixing ozone with the water (or other liquid A) destined for the exchanger 2, it is possible to effectively prevent infections by mycobacterium chimaera (and more besides), which otherwise could strike patients who are subjected to extracorporeal circulation, thus achieving the set aim.

The impurities, sediments, slime, biological structures and pathogens that gradually end up in suspension in the liquid A, while it flows through the components of the system 1 (the removal from the inner walls of these components is favored by the ozone), are collected and are retained by the filter 11, from which they can be removed at the end of the treatment (after having restored the service circuit 7a and then having removed the assembly 9).

It should be noted that the mixture of liquid A and ozone is typically (but not exclusively) kept heterogeneous, insofar as the ozone is also in the gaseous phase.

In particular, the connector 12 is arranged downstream of the filter 11. Such specific placement makes it possible to achieve further benefits: in this manner in fact, and as can be seen in Figure 1, the filter 11 is arranged directly upstream of the connector 12, at which the ozone is introduced into the temporary circuit 7b. In this manner, the mixture of water and ozone affects the filter 11 only after having almost completely passed through the temporary circuit 7b, and therefore after having accumulated the maximum quantity of impurities. The possibility is not ruled out however of arranging the connector 12 upstream of the filter 11, or in any case in any other position.

The connector 12 can be a "Y" connector of various types, and can be conveniently contoured so as to increase the speed of the gas by the Venturi effect, at its mixing with water (or other service liquid A).

Even more specifically, the filter 11 is an antibacterial filter and/or is constituted by a microporous filtering membrane (in any case the possibility of choosing further embodiments of the filter 11 comes under the scope of protection claimed herein).

Conveniently, in an embodiment of significant practical interest, which however does not limit the application of the invention, the filtering assembly 9 comprises an ozone diffuser 13, which is arranged along the tubular body 10 (and preferably but not exclusively made of ceramic).

The function of the diffuser 13 is to diffuse the ozone in the form of bubbles of very small average size, thus boosting the oxidant and disinfectant action (and the capacity to separate the slime and impurities from the inner walls of the conduits that it steadily flows over).

Furthermore, further useful contrivances can be adopted in order to increase the practicality and safety in use of the invention, such as for example one or more box-like shells 14, in which to insert the filtering assembly 9.

Attention is now drawn to the fact that the protection claimed herein relates not only to systems 1 according to what is described up to this point, but also to kits for preventing the proliferation of bacterial population, for service circuits 7a of extracorporeal circulation apparatuses, which comprise at least one unit 8 for dispensing ozone and/or a filtering assembly 9 according to what is noted up to this point.

According to this latter possibility, the protection is claimed of the supply and/or marketing of only the components that need to be used in substitution of the exchanger 2, in order to provide the temporary circuit 7b (the other components can be pre-existing).

In this manner in fact, as has been anticipated in the foregoing pages, the benefits associated with the application of the invention can apply to extracorporeal circulation apparatuses that are already installed in operating theaters (or elsewhere).

The object of the present discussion, and of the protection claimed herein, also relates to a method for preventing the proliferation of bacterial population, for service circuits 7a of extracorporeal circulation apparatuses. The service circuits 7a on which the method according to the invention is carried out are effectively of the type explained above. More generally, it must be noted that the method according to the invention can be carried out on systems 1 and/or by using kits according to the invention, as already described in the foregoing pages.

First of all therefore, the method according to the invention can be carried out on one or more service circuits 7a which are normally passed through by a service liquid A (preferably but not exclusively water). The service circuits 7a comprise at least one heat exchanger 2, which is passed through by blood destined for extracorporeal circulation (preferably, but not exclusively, cyclically), so that it can be heated and/or cooled by that service liquid A. Furthermore, the service circuits 7a comprise at least one tank 3 (or multiple tanks 3) for containing the liquid A, a delivery branch 4 and a return branch 6, with these branches interposed between the exchanger 2 and the tank 3. Preferably, the service circuits 7a on which the method according to the invention is carried out also include a pump 5, which is arranged along the delivery branch 4.

So according to the invention, the method consists first of all of a step a., of disconnecting the exchanger 2 from the branches 4, 6.

Subsequently, in a step b., the method entails connecting the tubular body 10 of a filtering assembly 9, which is provided with at least one filter 11, to the tank 3 via the branches 4, 6, so as to provide a temporary disinfection circuit 7b which comprises the tank 3, the tubular body 10 and the branches 4, 6.

The method then entails, in a step c., sending ozone to the tubular body 10, preferably at a connector 12 which is fitted on the body 10.

The method then consists of a step d., of circulating (for example, by way of the pump 5) the service liquid A, mixed with the ozone, in the temporary circuit 7b.

The activity d. can be prolonged for the length of time deemed necessary to adequately treat the liquid A, the tanks 3, the branches 4, 6 and any other component that may be comprised in the temporary circuit 7b. It is also possible that within the execution of the step d., the liquid can flow multiple times, cyclically, through the temporary circuit 7b.

At the end of the step d., the method according to the invention entails, in a step e., the disconnection of the tubular body 10 from the branches 4, 6 and the reconnection to the exchanger 2, in order to restore the service circuit 7a, thus making it available to the extracorporeal circulation apparatus with which it is intended to be used. It should be noted that the exchanger 2 will be passed through by water (or other service liquid A) which has previously been treated with ozone and therefore the risk of contact of the blood with water that is infected or otherwise rich in potentially hazardous microorganisms is guarded against, as is the risk of stagnation of vapor carrying microbial colonies.

It should be noted that the accompanying Figure 1 schematically shows the possibility of providing and connecting/disconnecting the circuits 7a, 7b: in this regard, it should be specified that the steps a., b., e. of connecting and disconnecting the exchanger 2 and of the body 10 can be carried out manually by an operator, just as the possibility is not ruled out of using automatic or semi-automatic solutions.

In particular, the step c. entails sending a predefined quantity of ozone to the tubular body 10, continuously, for a time comprised between 1 minute and 30 minutes, and preferably for a time comprised between 5 minutes and 10 minutes, and even more preferably for 5 minutes. Likewise, the predefined quantity of ozone to be sent in the period of time just defined is comprised between 50 mg and 150 mg and is preferably equal to 50 mg (although other values are not ruled out, for the duration of the step and/or the quantity of ozone). It should be noted that the step c. can be carried out using the previously-described unit 8, which is provided for example with a controller or other electronic device for control and management, which is preset and/or freely reconfigurable to dispense ozone according to the time and/or quantity parameters described above.

Whatever the duration of execution of the step c., it can be carried out in at least partial simultaneity with the step d., and therefore the dispensing of the ozone will be maintained while the pump 5 is already in operation, or in any case while the liquid A is moving in the temporary circuit 7b. Likewise, the scope of protection claimed herein covers the possibility that the step d. is carried out before starting step c.: in other words, moving the liquid A can begin even before introducing ozone into the temporary circuit 7b.

Conveniently, in order to ensure long-lasting conditions of hygiene and disinfection, the method according to the invention can preferably entail that the steps a., b., c., d. and e. (and therefore the disinfection treatment obtained by circulating ozone in the temporary circuit 7b) be repeated periodically according to a preset interval (for example every 1, 2 or 3 days at a scheduled time).

Operation of the system 1 and execution of the method according to the invention are clear from the description up to this point.

In any case, it should be noted that, in order to guard against the danger of infections in operating theaters where cardiac surgery operations are carried out that involve extracorporeal circulation apparatuses, it is possible to use the system 1 and/or the method according to the invention, by providing (preferably periodically, with a conveniently chosen interval) a temporary circuit 7b specifically for disinfecting the conduits through which flows the water (or other service liquid A) destined to exchange heat with the blood. In order to do this, the exchanger 2 is temporarily disconnected from the branches 4, 6 and these branches are connected to the filtering assembly 9, which in turn is associated with the unit 8 that dispenses ozone.

The ozone (in gaseous form) is injected into the temporary circuit 7b thus created, at the connector 12, is mixed with the water and from there it flows through the branches 4, 6 and each tank 3, as explained previously performing on these (and on the water) its oxidant and disinfectant action, on the mycobacterium chimaera and more besides.

After having left the ozone to act for the desired length of time, the assembly 9 is disconnected and the exchanger 2 is reconnected to the branches 4, 6, thus restoring the service circuit 7a which is now certainly free from pathogens (and also from other impurities that may be dissolved in water and/or accumulated over time along the inner walls of the conduits involved). It will moreover be possible to remove the filter 11 from the assembly 9 and/or in any case clean the filter, in order to free it from the residues and from the accumulated detritus, thus making it available for another use.

It should be noted that ozone is an unstable substance, and therefore its useful action ends (typically after about twenty to thirty minutes) when it is converted back to oxygen, without leaving harmful traces, if any, in the service circuit 7a and without causing any damage to the machines of the extracorporeal circulation apparatus.

It has therefore been shown that the system 1 and/or the method according to the invention ensure practical means of preventing infections by mycobacterium chimaera and of preventing the proliferation of bacterial population in general.

By virtue of the system and/or the method according to the invention it is therefore possible to sanitize and disinfect, simply and completely, conduits and machines involved in extracorporeal circulation.

In fact, ozone is first and foremost capable of treating and disinfecting the water that circulates along the temporary circuit 7b, and therefore along the service circuit 7a, once the latter has been restored for the activation of extracorporeal circulation. Furthermore, ozone can be liberated above the surface of the water in the tanks 3, performing its useful function on all the walls (including the ceiling) of these tanks, thus preventing or destroying the formation of colonies of bacteria in any part of the system 1. Even any stagnation of water vapor, as with the flow of water to the outside environment (for example the fall of droplets onto the floor of the operating theater), does not represent a threat to the health of individuals (and of patients in particular), since, having first been subjected to the circulation of ozone, the water is safe, being free from microorganisms (chimaera and/or others).

In order to obtain the important results shown above, a few simple activities are sufficient to provide and restore the circuits 7a, 7b, and this highlights the absolute simplicity and practicality of the invention, which can be easily put into practice by designated operators. It should be noted likewise that an entire disinfection cycle (a single iteration of the steps from a. to e. of the method according to the invention) can be completed in a short length of time (a few tens of minutes), and therefore without impacting significantly on the daily activities of personnel: this makes it possible to schedule a periodic repetition of the cycle, even with high frequencies, further ensuring prevention.

The invention thus conceived is susceptible of numerous modifications and variations, all of which are within the scope of the appended claims. Moreover, all the details may be substituted by other, technically equivalent elements.

In the embodiments illustrated, individual characteristics shown in relation to specific examples may in reality be substituted with other, different characteristics, existing in other embodiments.

In practice, the materials employed, as well as the dimensions, may be any according to requirements and to the state of the art.

The disclosures in Italian Patent Application No. 102019000004237 from which this application claims priority are incorporated herein by reference.

Where technical features mentioned in any claim are followed by reference signs, those reference signs have been included for the sole purpose of increasing the intelligibility of the claims and accordingly, such reference signs do not have any limiting effect on the interpretation of each element identified by way of example by such reference signs.

## Claims

1. A service system for extracorporeal circulation apparatuses, which comprises at least:
- a heat exchanger (2), which can be passed through by blood destined for extracorporeal circulation and by a service liquid (A) which is adapted to heat and/or cool the blood,
- a tank (3) for containing the service liquid (A),
- a delivery branch (4), normally interposed between said tank (3) and said exchanger (2), in order to send the liquid (A) to said exchanger (2),
- a return branch (6), normally interposed between said exchanger (2) and said tank (3), for the return of the liquid (A) to said tank (3),
said exchanger (2), said tank (3) and said branches (4, 6) forming a service circuit (7a), which is normally passed through by the service liquid (A),
**characterized in that** it comprises a unit (8) for dispensing ozone and a filtering assembly (9), which comprises a tubular body (10) which can be connected to said at least one tank (3) through said branches (4, 6) as a temporary replacement for said exchanger (2), to provide a temporary disinfection circuit (7b), said body (10) being affected by at least one filter (11) for the service liquid (A) and by a connector (12) for mixing the liquid (A) with the ozone dispensed by said unit (8).

2. The system according to claim 1, **characterized in that** said connector (12) is arranged downstream of said filter (11).

3. The system according to claims 1 and 2, **characterized in that** said filter (11) is an antibacterial filter.

4. The system according to one or more of the preceding claims, **characterized in that** said filter (11) is constituted by a microporous filtering membrane.

5. The system according to one or more of the preceding claims, **characterized in that** said filtering assembly (9) comprises an ozone diffuser (13) which is arranged along said tubular body (10).

6. The system according to one or more of the preceding claims, **characterized in that** said ozone generation unit (8) comprises an oxygen enrichment device, for the subsequent generation of ozone starting from atmospheric air.

7. A kit for preventing the proliferation of bacterial population, for service circuits (7a) of extracorporeal circulation apparatuses, which comprises at least one unit (8) for dispensing ozone and/or a filtering assembly (9) according to one or more of the preceding claims.

8. A method for preventing the proliferation of bacterial population, for service circuits (7a) of extracorporeal circulation apparatuses, said service circuits (7a) being normally passed through by a service liquid (A) and comprising at least one heat exchanger (2), which is passed through by blood destined for extracorporeal circulation for its heating and/or cooling by the service liquid (A), a tank (3) for containing the service liquid (A), a delivery branch (4) and a return branch (6), the branches (4, 6) being interposed between the exchanger (2) and the tank (3), the method consisting in:
a. disconnecting the exchanger (2) from the branches (4, 6),
b. connecting the tubular body (10) of a filtering assembly (9), which is provided with at least one filter (11), to the tank (3) via the branches (4, 6), in order to provide a temporary disinfection circuit (7b) which comprises the tank (3), the tubular body (10) and the branches (4, 6),
c. sending ozone to the tubular body (10),
d. circulating the service liquid (A), mixed with the ozone, in the temporary circuit (7b),
e. disconnecting the tubular body (10) from the branches (4, 6) and reconnecting the branches (4, 6) to the exchanger (2), in order to restore the service circuit (7a).

9. The method according to claim 8, **characterized in that** said step c. entails sending a predefined quantity of ozone to the tubular body (10), continuously, for a time comprised between 1 minute and 30 minutes, and preferably for a time comprised between 5 minutes and 10 minutes, and even more preferably for 5 minutes, the predefined quantity being comprised between 50 mg and 150 mg and being preferably equal to 50 mg.

10. The method according to claim 8 or 9, **characterized in that** said steps a., b., c., d. and e. are repeated periodically at preset intervals.
